## Europäisches Patentamt
## European Patent Office
## Office européen des brevets

(19)

(11) Veröffentlichungsnummer: **0 169 447**
**B1**

## EUROPÄISCHE PATENTSCHRIFT

(12)

(45) Veröffentlichungstag der Patentschrift:
**13.09.89**

(51) Int. Cl.⁴: **B 65 D 77/20,** A 61 B 19/02

(21) Anmeldenummer: **85108641.3**

(22) Anmeldetag: **11.07.85**

(54) Verpackung z. B. für chirurgische Instrumente.

(30) Priorität: **27.07.84 DE 3427702**

(43) Veröffentlichungstag der Anmeldung:
**29.01.86 Patentblatt 86/5**

(45) Bekanntmachung des Hinweises auf die Patenterteilung:
**13.09.89 Patentblatt 89/37**

(84) Benannte Vertragsstaaten:
**AT BE CH DE FR GB IT LI LU NL SE**

(56) Entgegenhaltungen:
**EP-A-0 034 551**
**DE-A-2 332 927**
**DE-A-2 717 866**

(73) Patentinhaber: **Dixie- Union Verpackungen GmbH,
Römerstrasse 12, D-8960 Kempten (DE)**

(72) Erfinder: **Hirt, Edmund, Zavelsteinstrasse 55,
D-7000 Stuttgart 30 (DE)**
Erfinder: **Send, Dietmar, Freudenthalstrasse 35,
D-8940 Memmingen (DE)**
Erfinder: **Diete, Günter, Schorenstrasse 17, D-8961
Buchenberg (DE)**

(74) Vertreter: **Pfister, Helmut, Dipl.- Ing., Buxacher
Strasse 9, D-8940 Memmingen/Bayern (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Verpackung z.B. für chirurgische Instrumente, wobei die Verpackung aus einer Kunststoffolienbahn und einer Papierbahn gebildet ist und die beiden Bahnen am Rand, der einen Aufnahmeraum umschließt, miteinander verbunden, insbesondere versiegelt sind, mit einer Ansatzstelle für einen Öffnungsvorgang, bei dem die Bahnen entlang des Randes voneinander abziehbar sind (siehe DE-A-2 332 927).

Es ist bekannt, z. B. chirurgische Instrumente, Spritzen, Katheter und ähnliche Waren steril zu verpacken, um zu erreichen, daß im Augenblick des Gebrauchs der verpackten Waren eine Sterilisation nicht mehr vorgenommen werden muß. Insbesondere ist dabei vorgesehen, daß die verpackten Waren nur zu einmaligem Gebrauch bestimmt sind, da davon ausgegangen wird, daß nur auf diese Weise ein Optimum an Sterilität erreichbar ist.

Nach der Verpackung der Waren, die in der Regel ohne Anwendung von Vakuum erfolgt, werden die fertigen Verpackungen einem Sterilisationsvorgang unterworfen. Die Anwendung von Dampf entsprechender Temperatur bzw. die Anwendung von Wärme zur Sterilisation ist dabei weniger zu bevorzugen, weil die erforderliche Wärme einen nachteiligen Einfluß sowohl auf die Verpackungsbestandteile, als auch auf die verpackten Gegenstände ausüben kann. Beispielsweise erweichen bei der Sterilisationstemperatur bereits die benützten Kunststoffbahnen oder die Kunststoffelemente der verpackten Waren. Bei vielen Medikamenten, Spritzen oder entsprechenden Waren verbietet sich ohnehin die Anwendung höherer Temperaturen.

Die Sterilisation erfolgt daher durch Begasung mit einem etwaige Keime abtötendem Gas. Zu diesem Zweck werden die Verpackungen in einen geschlossenen Behälter eingebracht und durch abwechselnde Anwendung von Unterdruck und Überdruck wird die Luft innerhalb der Verpackungen zu einem ausreichenden Teil durch das in den Behälter eingebrachte keimtötende Gas ersetzt.

Der Gasaustauschvorgang erfolgt dabei durch die Papierbahn hindurch, die entsprechend ausgebildet ist, derart, daß die Papierbahn zwar das Gas hinreichend passieren läßt, während etwaige Keime von der Papierbahn zurückgehalten werden.

Bis zum Gebrauch oder Verbrauch der Packungen und der verpackten Waren ergehen die Restgasmengen einen ausreichenden Schutz geben erneute Infektion, vorausgesetzt, daß die Verpackung ausreichend dicht ist.

Es bereitet nun an sich keine Schwierigkeiten, die Papierbahn und die Folienbahn derart miteinander zu verbinden, vorzugsweise zu versiegeln, um eine ausreichende Dichtheit zu gewährleisten. Der Rand, an dem die beiden Folienbahnen bei jeder Packung miteinander verbunden sind, muß hierfür nur eine entsprechende Breite besitzen, wobei in der Regel etwa 5 Milimeter als ausreichend angesehen werden. Durch einen geeigneten Siegelungsvorgang, der in der Regel unter Anwendung von Wärme erfolgt und wobei ein Siegelungswerkzeug auf der einen Seite den Rand gegen ein Widerlager drückt, das auf der anderen Seite angeordnet ist, läßt sich leicht ein dichter Verschluß erreichen.

Wenn nun eine derartig Verpackung geöffnet wird, so soll dies ohne die Anwendung zusätzlicher Werkzeuge möglich sein und es ist erwünscht, die beiden Bahnen, also die Kunststoffolienbahn und die Papierbahn voneinander abziehen zu können, wobei gegebenenfalls eine Anfasslasche oder eine andere Ansatzstelle für den Öffnungsvorgang benützt wird.

Die Bedingung dieser sogenannten "peel"-Fähigkeit setzt nun aber voraus, daß keine zu innige Verbindung zwischen den beiden Bestandteilen der Verpackung besteht. Haftet die Siegelung od. dgl. zu fest, läßt sich die Papierbahn nicht abziehen bzw. reißt durch, was den Öffnungsvorgang stört.

Weiter ergibt sich beim Abziehen der beiden Folien voneinander das Problem, daß bei zu inniger Haftung aus der Papierbahn Fasern und Faserbüschel ausgerissen werden, welche Fasern dann möglicherweise auf die verpackten Waren, also die chirurgischen Instrumente, fallen, was beispielsweise bei Operationen zu Entzündungen und anderen unerwünschten Folgen fahren kann. Es ist somit die Verbindung zwischen der Papierbahn und der Folienbahn so vorzunehmen, daß einerseits die "peel"-Fähigkeit erhalten bleibt und andererseits beim Abziehen auch keine losen Fasern entstehen.

Dabei muß aber beachtet werden, daß die Papierbahn in solcher Weise gestaltet ist, daß sie grundsätzlich gasdurchlässig ist, was die Anwendung von Mitteln, die geeignet sind, den Zusammenhalt des Faserverbandes der Papierbahn zu erhöhen, praktisch auschließt. Solche Mittel, wie Leim od. dgl., die sonst bei Papierbahnen zur Erhöhung der Festigkeit des Faserverbandes angewandt werden, würden die Gasdurchlässigkeit nachteilig beeinflussen.

Eine Verringerung der Haftwirkung der Siegelung zwischen den beiden Bahnen hat nun zur Folge, daß beim oben beschriebenen Gasaustausch, bei dem die Verpackung einem wiederholten Unterdruck und Überdruck ausgesetzt wird, die Gefahr besteht, daß bei der Anwendung von Unterdruck wobei sich die Verpackung aufbläht, die Siegelung beeinträchtigt wird, d.h. daß dann die Verpackung undicht ist. Derartige undichte Verpackungen können schwer von außen erkannt werden.

Wenn vorstehend oder nachstehend im Zusammenhang mit der Verbindung der Papierbahn mit der Kunststoffolienbahn von Siegelung gesprochen wird, so ist dies nicht als Beschränkung zu verstehen. Zwar wird eine Siegelung in der Regel angewandt werden, es ist aber auch möglich, die Siegelung durch einen Klebevorgang zu ersetzen. Auch Verbindungsmittel, die mehr als Verschweißung anzusehen sind, können angewandt werden.

Die Mittel, mit denen die Wirkung einer Siegelnaht vergrößert oder vermindert wird, sind an sich bekannt. In der DE-AS-2 608 777 wird für eine Verpackung ähnlicher Art jedoch zur Lösung einer anderen Aufgabe vorgeschlagen, die Schweißunterlage, die mit dem Versiegelungswerkzeug zusammenwirkt, so auszugestalten, daß sie eine Vielzahl von pyramidenförmigen Erhebungen aufweist, so daß sich eine Versiegelung ergibt, bei der die einzelnen Siegelelemente aus einer Vielzahl von Punkten bestehen.

Weiter wird in der genannten Druckschrift auch die Anwendung einer Schweißunterlage angeregt, die eine Vielzahl pyramidenförmiger Vertiefungen besitzt, so daß die Siegelelemente zwischen den beiden Bahnen eine netzartige Gestalt erhalten. Die Anwendung der beschriebenen Schweißunterlage erfolgt in der genannten Druckschrift zu dem Zweck, ein Anhaften der Verpackung an der Schweißunterlage zu vermeiden. Es ist jedoch gefunden worden, daß bei Anwendung der genannten Mittel dann, wenn die Ausdehnungen der einzelnen Siegelungspunkte oder Linien klein genug bleibt, die Haftwirkung der Siegelung gering ist, so daß eine gute "peel"-Fähigkeit erhalten wird.

Es ist ferner bekannt, den Rand der in Rede stehenden Verpackungen dadurch zu versiegeln, daß mehrere Siegelnähte im Rand angeordnet werden, wobei die Siegelnähte dann jeweils den gebildeten Aufnahmeraum umschließen. Die Anwendung einer Mehrzahl solcher parallel zum Rand verlaufenden linienförmigen Siegelnähte erhöht die Dichtheit und verbessert auch die Verbindung zwischen den beiden Bahnen. Beim Abziehen der einen Folie von der anderen ergibt sich aber unvermeidbar ein Ausfasern der Papierbahn mit allen nachteiligen Folgen.

Es ist Aufgabe der Erfindung, eine Verpackung der eingangs beschriebenen Gattung dahingehend zu verbessern, daß einerseits die absolute Dichtheit und der mechanische Zusammenhalt der beiden Bahnen gesichert bleibt, und daß andererseits die beiden Bahnen dennoch verhältnismäßig leicht voneinander abgezogen werden können, ohne daß die Gefahr besteht, daß aus der Papierbahn Fasern freigelegt werden.

Zur Lösung dieser Aufgabe geht die Erfindung aus von der Verpackung der eingangs beschriebenen Art und schlägt vor, daß der verbindende und zu öffnende Rand zwei gleichförmig verteilte Gruppen von Verbindungselementen, insbesondere Siegelungselementen, aufweist, von denen die erste Gruppe in mindestens einer Richtung in der Ebene des Randes vehältnismäßig kleine Abmessungen besitzt und die beiden Bahnen an viele Stellen dicht aufeinanderhält, und von denen die zweite Gruppe aus wesentlich längeren Linien begrenzter Breite besteht, die der mechanischen Verbindung der beiden Bahnen dienen wobei dies Linien derart ausgerichtet sind, daß sie quer zu der im wesentlichen vorgegebenen Abziehrichtung verlaufen.

Die Erfindung geht von der Überlegung aus, daß es grundsätzlich keinen nennenswert und zusätzlichen Aufwand bedeutet, wenn verschiedene Siegelungselemente zur Verbindung der beiden Bahnen angewandt werden. Die Siegelungselemente werden ja dadurch gewonnen, daß entweder die Siegelungsschiene oder die Schweißunterlage bzw. Gegenschiene hierzu oder beide Schienen eine geeignete Oberfläche aufweisen, die dann dafür sorgen, daß an der Verpackung die entsprechenden Siegelungselemente in der gewünschten Form und dadurch in der gewünschten Wirkung entstehen. In diesem Zusammenhang ist der Umstand von Bedeutung, daß es notwendig ist, die Anordnung derart zu treffen, daß beliebige Formen von Verpackungen gewonnen werden kennen. Die Siegelungsschienen und die Widerlager hierzu können bei der Erfindung ohne weiteres als plattenförmige Rohlinge hergestellt werden, aus denen dann beliebige Formen herausgearbeitet werden können, die den entsprechenden Verpackungsformen angepaßt sind.

Ist es nicht möglich, die Herstellung in dieser Weise zu bewirken, wird es notwendig, für jeden Verpackungstyp bzw. für jede Größe ein bestimmtes Werkzeug herzustellen. Da die Erfindung die verschiedenen Siegelungselemente gleichförmig verteilt, erreicht die Erfindung diesen wichtigen Vorteil.

Die Erfindung geht nun von der Überlegung aus, daß die erste Gruppe der Verbindungselemente so auszubilden ist, daß sie die Dichtheit gewährleisten und auch die "peel"-Fähigkeit sicherstellt, soweit es diese Siegelungselemente anbelangt. Die erste Gruppe hat in mindestens einer Richtung derart kleine Abmessungen, daß sie nicht in der Lage ist, Fasern aus dem Faserverband der Papierform auszureißen, weil die von diesen Siegelungselementen auf die Fasern übertragbaren Kräfte viel zu gering sind, um die Haftkräfte der Fasern im Faserverband zu überwinden. In aller Regel sind die Fasern wesentlich länger als den Abmessungen dieser Siegelungselemente entspricht, so daß beim Abziehen immer entweder die Verbindung zwischen den Fasern und Siegelungselementen getrennt wird, oder daß die Fasern, falls sie klein sind, zwar ausgezogen werden, jedoch dann an den Siegelungselementen haften.

Der zweiten Gruppe von Verbindungselementen bzw. Siegelungselementen kommt bei der Erfindung nun die Aufgabe zu, den mechanischen Zusammenhalt zwischen den beiden Bahnen sicherzustellen. Da diese Gruppe jedoch nicht die Aufgabe hat, beispielsweise eine ausreichende Dichtheit herbeizuführen, kann diese Gruppe entsprechend gestaltet werden.

Dies geschieht dadurch, daß die Linien nur eine begrenzte Breite aufweisen, die nicht ausreichen würde, eine Dichtheit herbeizuführen. Diese Gruppe von Siegelungselementen kann auch so ausgerichtet werden, daß beim Öffnungsvorgang keine nachteiligen Wirkungen auftreten. Dies erreicht die Erfindung dadurch, daß die Linien quer zu der vorgesehenen Abziehrichtung verlaufen. Würden die Linien im wesentlichen in der Abziehrichtung verlaufen, würden die linienförmigen Siegelungselemente beim Abziehen Faserreihen oder Faserbüschel ausziehen. Da die Linien aber quer zur Abzugsrichtung angeordnet sind, wird beim Abziehen nur eine verhältnismäßig geringe Breite der Papierbahn beeinträchtigt. Zwar werden Fasern ausgezogen, die annähernd parallel zur Linie verlaufen, wenn diese hinreichend mit den Siegelungselementen verbunden sind. Dies ist aber ohne Nachteil, da diese Fasern nicht frei werden. Fasern, die quer zur Linie verlaufen, werden dagegen nur geringfügig beansprucht.

In der Praxis hat sich gezeigt, daß es günstig ist, wenn die Linien nicht exakt rechtwinklig zur Abziehrichtung verlaufen, sondern mit der Abziehrichtung noch einen deutlichen spitzen Winkel einschließen. Dadurch ergibt sich eine Verminderung der jeweils beim Abziehen auftretenden Ausreißkräfte, was einerseits den Abziehvorgang vergleichmäßigt und andererseits die Gefahr der Bildung freier Fasern verringert.

In den Unteransprüchen sind einige bevorzugte Ausführungsformen der Erfindung gekennzeichnet.

In der Zeichnung sind einige Ausführungsbeispiele der Erfindung schematisch dargestellt. Es zeigen:

Fig. 1    eine Draufsicht auf eine Verpackung in erfindungsgemäßer Ausbildung,

Fig. 2    ein abgewandeltes Ausfürungsbeispiel der Erfindung,

Fig. 3    einen Schnitt durch die Darstellung der Fig. 1 entsprechend der Schnittlinie III - III und

Fig. 4    eine Draufsicht auf einen Teil einer anderen Ausführungsform der Erfindung in stark vergrößertem Maßstab.

Die in der Zeichnung gezeigte Verpackung besteht im wesentlichen aus der Unterfolie 9 aus Kunststoff, in die der becherartige Aufnahmeraum 10 eingeformt ist. Dieser nimmt die zu verpackenden Gegenstände 11 auf. Die aufgelegte Deckfolie 12 besteht aus Papier oder einem papierartigen Werkstoff. Am umlaufenden Rand 3 werden die Folien 9 und 12 miteinander verbunden, vorzugweise versiegelt. Zu diesem Zweck wirken, wie an sich bekannt, ein Siegelungswerkzeug und ein Gegenwerkzeug zusammen, die nicht näher dargestellt sind.

Eines oder beide Werkzeuge besitzen dabei eine Struktur zur Erzielung der Siegelungselemente, wie diese beschrieben worden sind und noch näher erläutert werden. Die Siegelung kann durch Beheizung eines der Werkzeuge erzielt werden oder auch durch Anwendung anderer Energieformen. Beide zusammenwirkenden Werkzeuge können hart und vergleichsweise unverformbar sein, also jeweils als metallische Werkzeuge ausgebildet sein. Es ist aber auch möglich, nur das Siegelungswerkzeug hart auszubilden, während das Gegenwerkzeug vergleichsweise nachgiebig ist. Ob das Siegelungswerkzeug von oben auf die Deckfolie 12 also auf die Papierbahn einwirkt oder von unten auf die Kunststofffolie, ist ohne wesentliche Bedeutung.

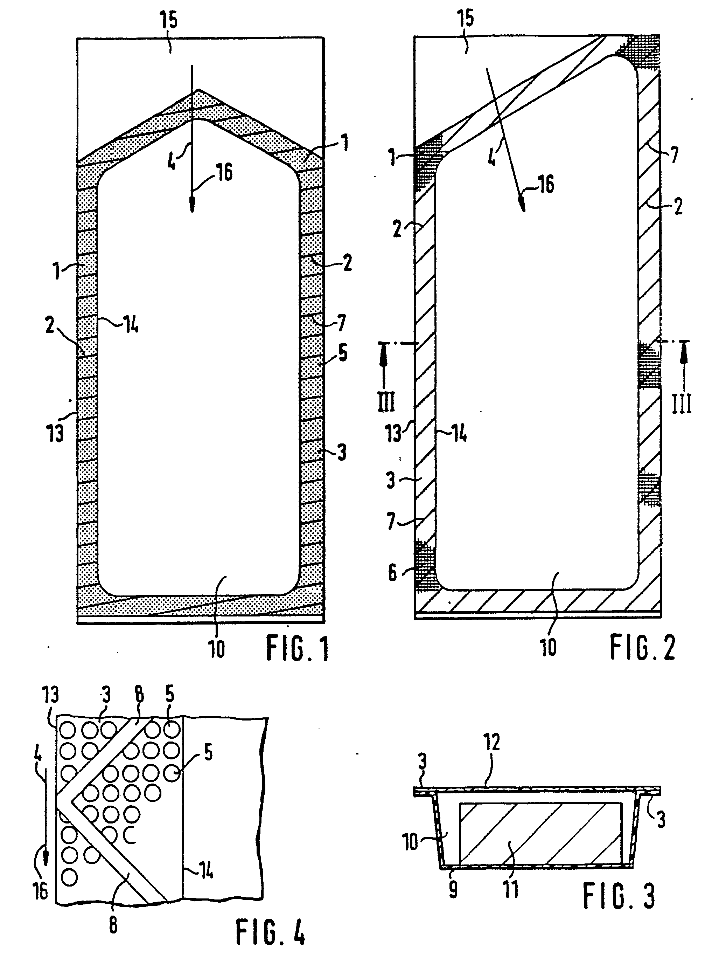

Der Rand 3 ist in der Regel von parallelen Konturen 13 und 14 begrenzt, wobei die Kontur 13 im wesentlichen den Außenrand der Packung bildet, während die Kontur 14 gleichzeitig den Aufnahmeraum 10 begrenzt. Der Rand kann umlaufend gleich breit sein. In der Regel reicht eine Breite vor etwa 5 Milimetern aus. Bei den gezeigten Ausführungsbeispielen nach den Fig. 1 und 2 umschließt der Rand 3 jeweils den Aufnahmeraum 10 vollständig und ist auch in allen Teilen im wesentlichen gleichartig ausgebildet. Die Erfindung ist aber auch anwendbar, wenn beispielsweise der untere Teil des Randes 3 nicht abziehfähig ist und beim öffnen der Verpackung auch insofern keine Trennung erfolgt.

Beim Ausführungsbeispiel der Fig. 1 kann die Verpackung im Bereich der Ansatzstelle 15 ergriffen werden, wo die beiden Bahnen bzw. die Unterfolie und die Deckfolie nicht miteinander verbunden sind.

Die Deckfolie, also die Papierbahn kann in Richtung des Pfeiles 16 herabgezogen werden.

Da, wie in der Fig. 1 gezeigt, die erste Gruppe 1 der Siegelungselemente als Punkte 5 ausgebildet sind, lösen sich diese Siegelungselemente leicht ohne den Faserverband der Papierbahn zu beeinträchtigen. Die Linien 7, die quer zur Richtung des Pfeiles 16 verlaufen und die die zweite Gruppe 2 der Siegelungselemente bilden, erhöhen zwar beträchtlich den Zusammenhang zwischen den beiden Bahnen, ohne beim Abziehen die Fasern aus der Deckfolie 12 auszureißen.

Das Ausführungsbeispiel nach der Fig. 2 unterscheidet sich dadurch vom Ausführungsbeispiel nach der Fig. 1, daß die Ansatzstelle 15 seitlich angeordnet ist, so daß sich hierdurch eine etwas andere Abziehrichtung ergibt. Beim Ausführungsbeispiel nach der Fig. 2 sind die Siegelungselemente der ersten Gruppe in Form eines Netzes 6 gestaltet, das aber sehr dünne Linien besitzt.

Die Fig. 4 zeigt punktförmige Siegelungselemente 5 in Kombination mit zickzackförmigen Linien 8. Die Abziehrichtung 4 ist durch den Pfeil 16 angedeutet.

Der Abstand der Punkte 5 voneinander und die Größe der Punkte ist so zu wählen, daß nur eine dichte Verbindung erhalten nicht jedoch eine flächig Versiegelung erreicht wird.

Bei einem Abstand der Punkte von weniger als einem Milimeter und einem Punktdurchmesser in der Größe eines Bruchteils hiervon, werden brauchbare Ergebnisse erzielt. Der Abstand der Linien des Netzes 6 voneinander kann etwa dem Abstand der Punkte 5 voneinander entsprechen. Die Breite der Netzteile muß jedoch hinreichend gering sein, um eine flächenartige Haftung die nennenswerte Kräfte übertragen könnte, zu vermeiden.

Im Gegensatz zu den Siegelungselementen der Gruppe 1 sind die Siegelungselemente 7 und 8 länger und erstrecken sich über die Breite des Randes 3. Die Breite dieser Siegelungselemente 7 und 8 sollte jedoch den Betrag von etwa 1 Millimeter nicht wesentlich überschreiten.

Die Zickzacklinien 8 des Beispiels nach der Fig. 4 können beispielsweise Teile eines Gitters sein. Die Eckpunkte der Zickzacklinien müssen dabei im Rand 3 nicht wiedererscheinen.

Die Erfindung läßt sich auch verwirklichen, wenn die Siegelungselemente andere, als die gezeigten Formen, aufweisen. So können die Siegelungselemente 1 anstelle von kreisförmigen Punkten auch längliche Gebilde sein. Die Siegelungselemente der Gruppe 2 können auch von Wellenlinien gebildet sein, wobei darauf zu achten ist, daß Teilabschnitte parallel zur Abziehrichtung vermieden werden.

## Patentansprüche

1. Verpackung z. B. für chirurgische Instrumente, wobei die Verpackung aus einer Kunststoffolienbahn (9) und einer Papierbahn (12) gebildet ist und die beiden Bahnen (9, 12) am Rand (3), der einen Aufnahmeraum (10) umschließt, miteinander dicht verbunden, insbesondere versiegelt sind, mit einer Ansatzstelle (15) für einen Öffnungsvorgang, bei dem die Bahnen entlang des Randes voneinander abziehbar sind, dadurch gekennzeichnet, daß der verbindende und zu öffnende Rand (3) zwei gleichförmig verteilte Gruppen von Verbindungselementen, insbesondere Siegelungselementen (1, 2) aufweist, von denen die erste Gruppe (1) in mindestens einer Richtung in der Ebene des Randes (3) verhältnismäßig kleine Abmessungen besitzt und die beiden Bahnen (9, 12) an vielen Stellen dicht aufeinander hält, und von denen die zweite Gruppe (2) aus wesentlich längeren Linien begrenzter Breite besteht, die der mechanischen Verbindung der beiden Bahnen (9, 12) dienen, wobei die Linien derart ausgerichtet sind, daß sie quer zu der im wesentlichen vorgegebenen Abziehrichtung (4) verlaufen.

2. Verpackung nach Anspruch 1, dadurch gekennzeichnet, daß die erste Gruppe (1) von Siegelungselementen punktförmig gestaltet sind.

3. Verpackung nach Anspruch 1, dadurch gekennzeichnet, daß die erste Gruppe (1) von Siegelungselementen (6) ein Netz bilden.

4. Verpackung nach einem oder mehreren der vorhergehenden Ansprüche, dadurch gekennzeichnet, daß die zweite Gruppe (2) von Siegelungselementen (7) aus einer Schar paralleler Linien besteht, die mit der Begrenzung des Randes (3) spitze Winkel einschließen.

5. Verpackung nach Anspruch 4, dadurch gekennzeichnet, daß der spitze Winkel etwa 45° beträgt.

6. Verpackung nach einem der Ansprüche 1 - 3, dadurch gekennzeichnet, daß die zweite Gruppe (2) von Siegelungselementen von Zickzacklinien gebildet ist.

## Claims

1. Package, for example for surgical instruments, formed from a strip of plastic (9) and a strip of paper (12), and the two strips (9, 12) are joined, especially sealed, at the edge (3), which surrounds a receptive cavity (10), having a tab zone (15), where the two strips can be peeled off along the edge characterized in that the bonded edge (3) to be opened has two uniformly distributed groups of bonding elements, especially sealing elements (1, 2), of which the first group (1) is of relatively small dimensions in at least one direction in the plane of the edge (3) and holding the two strips closely together at many places, and of which the second group (2) consists of considerably longer lines of limited width, serving the mechanical cohesion of the two strips (9, 12), whereby the lines are arranged in such a way that they run transversely to the predetermined direction of peel (4).

2. A packaging device as claimed in claim 1 characterized in that the elements of the first group (1) of sealing elements are in the form of points.

3. A packaging device as claimed in claim 1 characterized in that the first group (1) of sealing elements form a net.

4. A packaging device as claimed in one or more claims mentioned before characterized in that the second group (2) of sealing elements (7) is formed by a crowd of parallel lines (7), where the lines are at an acute angle with the border of the edge (3).

5. A packaging device as claimed in claim 4 characterized in that the acute angle is about 45 degrees.

6. A packaging device as claimed in the claims 1 - 3 characterized in that the second group (2) of sealing elements is formed by zig-zag lines.

## Revendications

1. Emballage, par exemple pour instruments chirurgicaux, l'emballage étant constitué par une bande de feuille de matière plastique (9) et une bande de papier (12) et les deux bandes (9, 12) étant reliées, en particulier scellées, au bord (3) qui entoure un volume de réception (10), avec un appendice (15) pour une opération d'ouverture au cours de laquelle les bandes peuvent être décollées l'une de l'autre le long du bord, caractérisé en ce que le bord (3) à sceller et à ouvrir comprend deux groupes d'éléments de liaison, en particulier d'éléments de scellement (1, 2) répartis uniformément dont le premier groupe (1) présente des dimensions relativement faibles

9 EP 0 169 447 B1 10

dans au moins l'une des directions dans le plan du bord (3) et assure en un grand nombre de points un contact étanche entre les deux bandes (9, 12), et dont le second groupe (2) est constitué de lignes beaucoup plus longues d'une largeur réduite qui sont destinées à assurer la liaison mécanique des deux bandes (9, 12), les lignes étant orientées de telle façon qu'elles s'étendent transversalement à la direction de décollement (4) fondamentalement prédéterminée.

2. Emballage selon la revendication 1, caractérisé en ce que le premier groupe (1) d'éléments de scellement est conformé en points.

3. Emballage selon la revendication 1, caractérisé en ce que le premier groupe (1) d'éléments de scellement (6) forme un réseau.

4. Emballage selon l'une ou plusieurs des revendications précédentes, caractérisé en ce que le second groupe (2) d'éléments de scellement (7) se compose d'une famille de lignes (7) parallèles qui renferment avec la délimitation du bord (3) des angles aigus.

5. Emballage selon la revendication 4, caractérisé en ce que l'angle aigu est d'environ 45°.

6. Emballage selon l'une des revendications 1 à 3, caractérisé en ce que le second groupe (2) d'éléments de scellement est constitué par des lignes en zig-zag.

FIG. 1

FIG. 2

FIG. 4

FIG. 3